# EUROPÄISCHE PATENTSCHRIFT

(11) **EP 3 184 078 B1**
(45) Veröffentlichungstag und Bekanntmachung des Hinweises auf die Patenterteilung: **07.10.2020**
(21) Anmeldenummer: 16204600.7
(22) Anmeldetag: 16.12.2016
(51) Int. Cl.: A61F 2/08, A61B 17/064, A61B 17/80

(54) **SEHNEN - FIXATIONSPLATTE**
TENDON FIXATION PLATE
PLAQUE DE FIXATION DE TENDONS

(30) Priorität: 23.12.2015 DE 102015122730
(43) Veröffentlichungstag der Anmeldung: 28.06.2017
(73) Patentinhaber: INOVEDIS GmbH, 72461 Albstadt (DE)
(72) Erfinder: Flöß, Lukas, 72519 Veringenstadt (DE); Oblak, Peter, 72458 Albstadt (DE); Welte, Stefan, 72458 Albstadt (DE)
(74) Vertreter: Otten, Roth, Dobler & Partner mbB Patentanwälte

(56) Entgegenhaltungen:
- WO-A1-00/64365
- WO-A1-02/34166
- FR-A1- 2 725 126
- FR-A1- 2 980 966
- US-A- 5 314 427
- US-A1- 2010 145 386
- US-A1- 2015 230 839

## Beschreibung

Die Erfindung betrifft ein Implantat zur flächigen Verbindung von Gewebe mit Knochen gemäß Anspruch 1 sowie ein System zum Verbinden von Gewebe mit Knochen, umfassend wenigstens ein Implantat und wenigstens ein Werkzeug gemäß Anspruch 11.

Verschiedene Umstände können dazu führen, dass man einem operativen Eingriff zur Fixierung und Rekonstruktion von abgerissenen Geweben, z.B. Sehnengewebe, unterliegen muss. Aus dem Stand der Technik ist diesbezüglich bekannt, dass zur Rekonstruktion beispielsweise einer Sehne deren abgetrenntes Ende am Knochen mittels eines Implantates fixiert wird. Dem Fachmann ist bezogen auf das Implantat auch der Begriff Anker bzw. Knochenanker geläufig. Beispielhaft ist ein derartiger Anker in der EP 2 581 046 A1 offenbart. Üblicherweise wird ein solcher Anker in den Knochen zwecks Befestigung eingetrieben. Dies erfordert in aller Regel eine entsprechende Vorbehandlung des Knochens, beispielsweise eine geeignete Bohrung für den Anker bereitzustellen. Mittels eines Nahtfadens, der mit dem Anker verbunden ist bzw. wird, erfolgt eine Fixation des Gewebes, beispielsweise der Sehne derart, dass das Gewebeende mittels Nahtfaden angeschlungen wird und so am Knochen zur Einheilung fixiert wird. Nachteilig ist hierbei insbesondere, dass ein erhöhter Zeitaufwand und damit einhergehend ein erhöhter Kostenaufwand bei der Fixierung der Sehne entsteht.

Zudem ist von Nachteil, dass ggf. keine ausreichende Stabilität bei der Fixierung der Sehne erzielt wird, beispielsweise aufgrund eines fehlerhaften Ausführens während der Fixation. Aus dem Stand der Technik der EP 0 852 128 B1 ist eine chirurgische Klammer zur Verbindung zweier Knochenteile oder zur Verbindung von Sehnen bzw. Bändern mit Knochenteilen bekannt, die zwei Befestigungsmittel aufweist. Hierbei ist ein Befestigungsmittel beispielsweise im Bereich eines Knochenteiles und ein Befestigungsmittel beispielsweise im Bereich einer Sehne bzw. eines Bandes angeordnet. Von Nachteil ist hierbei, dass eine derartige Verbindung unter Umständen keine ausreichende Stabilität aufweist bzw. ein Lösen des Gewebes vom Knochen stattfindet, so dass ein erneuter chirurgischer Eingriff bei einem solchen Lösen nötig ist.

Die FR 2 980 966 A1 offenbart eine medizinische Klammer, wobei die Klammer Hakenelemente aufweist, die durch eine mechanische Verbindung verstellbar miteinander verbunden sind, um während eines Aufpralls der Laschen in die Knochenkörper gleiten zu können.

Eine fusionsfreie Methode zur Korrektur von Wirbelsäulenverformungen bei wachsenden Jugendlichen wird unter Verwendung einer in der WO 00/64365 A1 beschriebenen Formgedächtnislegierungsheftklammer offenbart.

In der US 2010/145386 A1 ist eine plastisch verformbare Wirbelsäulenstabilisierungsplatte beschrieben, welche eine Anzahl von Schraub-Halte-Steckdosen aufweisen kann, um die Befestigung an der Wirbelsäule zu erleichtern.

Weiterhin kennt man aus der US 5 314 427 A eine implantierbare Kanalbandklemme zur Verbindung von Gewebe mit Knochen.

Aus der US 2015/230839 A1 kennt man bereits ein als medizinische Klammer ausgebildetes Implantat gemäß dem Oberbegriff des Anspruchs 1. Nachteilig an dem bekannten Implantat ist, dass eine Befestigung von Gewebe an Knochen und umgekehrt lediglich über an einer Klemmfläche angeordneten Befestigungsmitteln erfolgt. Somit kann eine dauerhafte bzw. stabile Befestigung von Gewebe und Knochen, insbesondere bei erhöhten Belastungen, nicht gewährleistet werden.

Aufgabe der Erfindung ist es daher, eine auch bei erhöhten Belastungen dauerhafte bzw. stabile Befestigungsmöglichkeit von Gewebe mit Knochen im Rahmen eines operativen Eingriffs unter vermindertem Arbeits- und Zeitaufwand bereitzustellen, welche zudem nekrotische Prozesse minimieren kann.

Zur Lösung der Aufgabe wird ein Implantat zur flächigen Verbindung von Gewebe mit Knochen vorgeschlagen, wobei das Implantat eine Klemmfläche und wenigstens drei mit der Klemmfläche verbundene Befestigungsmittel umfasst. Dadurch wird es ermöglicht, Gewebe mit Knochen fadenfrei zu verbinden. Die Verbindung von Gewebe und Knochen erfolgt dabei insbesondere über die Klemmfläche. Somit erfolgt ein flächiges Verbinden von Gewebe mit Knochen bzw. ein Anpressen des Gewebes auf den Knochen. Der benötigte Zeitaufwand kann dadurch bei einem operativen Eingriff signifikant reduziert werden. Wenigstens drei Befestigungsmittel sorgen dazu für ausreichenden Halt des Implantates. In vorteilhafter Weise kann somit auch eine Behandlungsdauer nach einem operativen Eingriff reduziert werden, da Nachbehandlungen deutlich erleichtert werden können. Hierdurch können auch Behandlungskosten reduziert werden.

Die Klemmfläche umfasst zudem einen äußeren Rand und einen inneren Rand, wobei der äußere Rand zumindest teilweise mittels wenigstens eines Verbindungssteges zumindest teilweise mit dem inneren Rand verbunden ist und/oder die Klemmfläche umfasst einen äußeren Rand und wenigstens eine innerhalb der Klemmfläche angeordnete Öffnung, welche Öffnung mittels wenigstens eines Verbindungssteges zumindest mit einem Teilbereich des äußeren Randes der Klemmfläche verbunden ist.

In vorteilhafter Weise kann eine Klemmfläche, die einen äußeren Rand, einen inneren Rand sowie Verbindungsstege bzw. Öffnungen umfasst, nach einem Einbringen des Implantates das Voranschreiten nekrotischer Prozesse bei einer flächigen Verbindung von Gewebe und Knochen unterbinden. Etwaige Behandlungen nach einem Einbringen des Implantates bzw. einem Verbinden von Gewebe und Knochen können dadurch ausbleiben.

Vorteilhaft ist zudem, dass punktuelle Druckspitzen vermieden werden, wodurch eine suffiziente Durchblutung gewährleistet werden kann. Zudem kann durch das Implantat ein kontinuierlicher Anpressdruck erreicht werden, der über den gesamten Zeitraum einer Einheilung aufrecht erhalten werden kann. Insofern kann eine zügigere Einheilung ermöglicht werden.

Erfindungswesentlich ist, dass die Klemmfläche wenigstens einen Ausleger umfasst, der wenigstens eine Öffnung aufweist und dass die Klemmfläche wenigstens ein Fixiermittel umfasst, das vorzugsweise an einer Unterseite angeordnet ist. Dadurch kann eine Fixierung der Klemmfläche auf dem Gewebe ermöglicht werden. Dies kann die Befestigung von Gewebe mit Knochen vereinfachen und die Verbindung von Gewebe und Knochen verstärken. Dadurch kann eine weitere Befestigungsmöglichkeit des Implantates bereitgestellt werden. Das Einbringen des Implantates bei einem operativen Eingriff kann zudem vereinfacht werden. Beispielsweise kann ein weiteres Befestigungsmittel herangezogen werden, welches in der Öffnung des Auslegers angeordnet ist und Gewebe und Knochen miteinander verbindet. Es kann auch ermöglicht werden, dass das Implantat mit Gewebe und zusätzlich mit dem Knochen befestigbar ist.

In einer Weiterbildung kann vorgesehen sein, dass wenigstens ein Befestigungsmittel einstückig an der Klemmfläche angeformt ist und/oder dass zumindest ein Befestigungsmittel kegelförmig ausgebildet ist und/oder dass das wenigstens eine Befestigungsmittel wenigstens einen Widerhaken umfasst. Einstückig an der Klemmfläche angeformte Befestigungsmittel können die Stabilität bzw. den Halt des angebrachten Implantates erhöhen. Widerhaken am Befestigungsmittel können einen festen Halt des Implantates begünstigen. Ist ein Befestigungsmittel kegelförmig ausgebildet, kann dies ein Einbringen des Implantates während eines operativen Eingriffs beschleunigen.

In einer Weiterbildung kann vorgesehen sein, dass wenigstens zwei Befestigungsmittel in etwa parallel zueinander angeordnet sind und/oder unterschiedliche Längen aufweisen und/oder dass die Klemmfläche eine Ebene bildet, wobei wenigstens ein Befestigungsmittel in etwa senkrecht zu der Ebene und/oder wenigstens ein Befestigungsmittel in etwa parallel zu der Ebene angeordnet ist. Durch die unterschiedliche Ausbildung bzw. Anordnung der Befestigungsmittel können individuelle

anatomische Gegebenheiten berücksichtigt werden. Daraus kann ein reduzierter Zeitaufwand bei einem operativen Eingriff resultieren. Daraus kann weiterhin ein verbesserter Halt des Implantates und damit einhergehend eine feste Verbindung zwischen Gewebe und Knochen resultieren.
In einer vorteilhaften Weiterbildung kann vorgesehen sein, dass die Klemmfläche eine Werkzeugangriffsfläche umfasst. Dadurch kann ein Werkzeug direkt an dem Implantat angesetzt werden, um das Implantat einzubringen bzw. um Gewebe und Knochen miteinander zu verbinden. Dadurch kann der Zeitaufwand zum Verbinden von Knochen und Gewebe signifikant reduziert werden.

Erfindungsgemäß kann vorgesehen sein, dass der Ausleger eine Länge aufweist, die zwischen 5 mm und 20 mm, vorzugsweise zwischen 6 und 13 mm liegt. Besonders vorteilhaft ist, wenn der Ausleger eine Länge aufweist, die 6,5 mm oder 12,5 mm beträgt.

Eine Weiterbildung kann vorsehen, dass die Klemmfläche wenigstens einen Ausleger umfasst, wobei der wenigstens eine Ausleger etwa entlang einer Quererstreckung der Klemmfläche angeordnet ist und/oder dass der wenigstens eine Ausleger und die Klemmfläche einstückig ausgestaltet sind. Dies stellt eine weitere Befestigungsmöglichkeit des Implantates, beispielsweise am Knochen dar. Dadurch kann ermöglicht werden, dass die Verbindung zwischen Gewebe und Knochen nach einem Einbringen des Implantates erhöhten Belastungen standhält. Somit können Behandlungen nach einem Verbinden von Gewebe und Knochen mittels des Implantates ausbleiben.

Eine bevorzugte Ausgestaltung kann vorsehen, dass die Klemmfläche wenigstens zwei sich jeweils konisch verjüngende Langlöcher umfasst, die diametral angeordnet sind. Es kann somit ermöglicht werden, dass ein Werkzeug mittels einer Drehbewegung einbringbar und/oder ausbringbar ist. Ein derartiges Zusammenwirken von Werkzeug und Implantat, das ein System darstellen kann, kann als Bajonett-Verschluss aufgefasst werden. Es kann dadurch ermöglicht werden, das Einbringen des Implantates bei einem operativen Eingriff zu vereinfachen und zu beschleunigen. Zudem können die Langlöcher in der Klemmfläche nach einem Einbringen des Implantates nekrotische Prozesse reduzieren, da die Langlöcher als Aussparung bzw. Öffnung in der Klemmfläche angesehen werden können.

Im Sinne der Erfindung kann der Begriff Aussparung synonym zu dem Begriff Öffnung angesehen werden.

In einer Weiterbildung kann vorgesehen sein, dass die Klemmfläche einen zumindest abschnittsweise gerundeten Umfang aufweist und/oder zumindest abschnittsweise gewölbt ist. Es ist somit möglich, individuelle anatomische Gegebenheiten zu berücksichtigen. Dadurch kann das Einbringen des Implantates bzw. das Verbinden von Gewebe und Knochen vereinfacht bzw. beschleunigt werden. Zudem kann ein stabiler Halt von Gewebe und Knochen erreicht werden. Es wird dadurch ferner ein gleichmäßiges Anpressen des Gewebes auf der gesamten Fläche der Klemmfläche erreicht. Vorteilhaft ist bei einer gewölbten Form der Klemmfläche, dass ein einfaches anmodellieren des Implantates am Knochen ermöglicht werden kann.

In besonders vorteilhafter Weise kann vorgesehen sein, dass die Klemmfläche einen im Wesentlichen ovalen Umfang aufweist. Auf diese Weise kann eine möglichst große Auflagefläche bzw. Klemmfläche des Implantates bereitgestellt werden.

Es kann vorgesehen sein, dass die Klemmfläche eine Längserstreckung aufweist, die in einem Bereich zwischen 2 mm und 30 mm, vorzugsweise zwischen 5 mm und 20 mm liegt und/oder dass die Klemmfläche zumindest in einem überwiegenden Teilbereich eine Dicke aufweist, die zwischen 0,2 mm und 4 mm, vorzugsweise zwischen 0,3 mm und 3 mm liegt und/oder dass zumindest die Klemmfläche aus einem metallischen Werkstoff, insbesondere Titan oder aus einem Kunststoff, insbesondere aus einem biokompatiblen Kunststoff gefertigt ist. Eine Klemmfläche, die aus einem metallischen Werkstoff, insbesondere Titan oder aus einem Kunststoff, insbesondere aus einem biokompatiblen Kunststoff gefertigt ist, weist einerseits eine ausreichende Stabilität auf, um Gewebe und Knochen miteinander zu verbinden. Andererseits sind derartige Materialien für den menschlichen Körper gut verträglich. Längserstreckungen der Klemmfläche, die in einem Bereich zwischen 2 mm und 30 mm, vorzugsweise zwischen 5 mm und 20 mm liegen, sind besonders vorteilhaft für die Verbindung von Sehnen und Knochen, insbesondere im Bereich der menschlichen Schulter. Dies gilt gleichermaßen für Dicken, die zwischen 0,2 mm und 4 mm, vorzugsweise zwischen 0,3 mm und 3 mm liegen. Besonders vorteilhaft ist, wenn die Klemmfläche des Implantates 20 mal 15 mm oder 14 mal 10 mm beträgt. Die Angaben beziehen sich dabei auf Länge mal Breite bzw. Längserstreckung und Quererstreckung. Derartige Klemmflächen können eine besonders sichere bzw. stabile Fixierung von Gewebe auf Knochen ermöglichen.

Die Fixiermittel können erfindungsgemäß auch als so genannte Spikes bezeichnet werden. Diese können ein Durchrutschen des Gewebes, beispielsweise einer Sehne, verhindern.

Eine Weiterbildung der Erfindung kann vorsehen, dass das Implantat eine im Wesentlichen in viereckiger Form ausgebildete Klemmfläche umfasst, an welcher wenigstens vier einstückig angeformte Befestigungsmittel angeordnet sind.

Es kann weiterhin vorgesehen sein, dass ein Ausleger wenigstens ein Fixiermittel umfasst. Dadurch kann die Verbindung von Gewebe und Knochen weiter verstärkt werden. Erfindungsgemäß kann vorgesehen sein, dass ein Verhältnis der der Summe der Flächen der Öffnungen der Klemmfläche und der Langlöcher zu der Gesamtfläche der Klemmfläche 30% bis 70%, insbesondere 40% bis 60% betragen.

Erfindungsgemäß kann auch vorgesehen sein, dass etwa 50% der Gesamtfläche der Klemmfläche Öffnungen aufweisen. Dies kann Langlöcher mit einschließen.
Es kann somit ein optimiertes Verhältnis zwischen Öffnungen und Gesamtfläche bereitgestellt werden, d.h. ein optimiertes Verhältnis zwischen Auflagefläche und somit Befestigung des Implantates sowie Öffnungen bzw. Ermöglichen von Durchblutung des Gewebes und somit Minimierung nekrotischer Prozesse.

Die Erfindung kann auch ein Implantat vorsehen, bei dem die Klemmfläche einen im Wesentlichen viereckigen Umfang aufweist und welches vier einstückig an jeweils an einer Ecke der Klemmfläche angeformte Befestigungsmittel umfasst. Ein derartiges Implantat kann eine besonders stabile Befestigung von Gewebe und Knochen ermöglichen. Ein derartiges Implantat kann beispielsweise jedoch nicht ausschließend im Bereich der Veterinärmedizin zum Einsatz kommen.

Die Erfindung kann zudem ein Implantat vorsehen, das eine im Wesentlichen runde, insbesondere ovale Form aufweist, wobei das Implantat vier einstückig an der Klemmfläche angeordnete Befestigungsmittel umfasst.

Bevorzugt kann hierbei vorgesehen sein, dass die Klemmfläche Längen von 6 mm bis 24 mm und Breiten von 4 mm bis 22 mm aufweist. Insbesondere ist vorgesehen, dass die Klemmfläche Maße von 6 mm Länge und 8 mm Breite oder von 10 mm Länge und 8 mm Breite oder von 12 mm Länge und 10 mm Breite oder von 14 mm Länge und 12 mm Breite oder von 16 mm Länge und 14 mm Breite oder von 18 mm Länge und 16 mm Breite aufweist. Die Erfindung betrifft auch ein System zum Verbinden von Gewebe mit Knochen, umfassend wenigstens ein Implantat und wenigstens ein Werkzeug, wobei das Werkzeug ein Griffmittel und einen daran drehfest angeordneten Schaft umfasst, wobei das Werkzeug ein Mitnahmeprofil umfasst, wobei das Mitnahmeprofil einen Sockel und wenigstens zwei Haltemittel umfasst und wobei das Implantat eine Klemmfläche und wenigstens drei mit der Klemmfläche verbundene Befestigungsmittel umfasst.

Erfindungsgemäß kann weiterhin vorgesehen sein, dass bei dem System das Implantat gemäß den offenbarten Merkmalen ausgebildet ist.

Erfindungsgemäß kann vorgesehen sein, dass das Mitnahmeprofil einen Sockel und wenigstens zwei Haltemittel umfasst. Dadurch wird ein Halt des Werkzeugs an einem Implantat und zudem eine Positionierung des Werkzeugs ermöglicht. Somit kann das Werkzeug für ein besonders zeiteffizientes Einbringen eines Implantats zur Verbindung von Gewebe mit Knochen herangezogen werden.

In einer Weiterbildung kann vorgesehen sein, dass die wenigstens zwei Haltemittel mit dem Sockel verbunden sind und/oder das Mitnahmeprofil einen Sockel umfasst, der einen größeren Umfang als ein Umfang des Schafts aufweist und/oder einstückig mit dem Schaft verbunden ist. Mit dem Sockel verbundene Haltemittel können eine Erhöhung der Stabilität des Werkzeugs bewirken. Dadurch kann ein sicheres und vollständiges das Einbringen des Implantates ermöglicht werden. Dies gilt gleichermaßen für den Fall, dass der Sockel einen größeren Umfang als ein Umfang des Schafts aufweist. Hieraus kann eine verbesserte Kraftverteilung auf das Implantat während des Einbringens des Implantates resultieren.

Es kann vorgesehen sein, dass der Sockel und der Schaft einstückig ausgestaltet sind und/oder die wenigstens zwei Haltemittel vorzugsweise als Nasen ausgebildet sind und einen im Wesentlichen runden oder polygonen Umfang aufweisen. Ein derartiges Werkzeug ist kostengünstig produzierbar und robust, wodurch auch ein gleichmäßiges und effizientes Einbringen des Implantates ermöglicht werden kann.

Die Erfindung kann auch vorsehen, dass der Sockel und der Schaft lösbar verbindbar sind. So kann ermöglicht werden, ein vielseitigeres Werkzeug mit einem Schaft bereitzustellen. Es ist somit möglich, unterschiedliche Sockelausgestaltungen mit einem Werkzeug zu verbinden.

Eine derartige lösbare Verbindung von Sockel und Schaft kann beispielsweise mit einem Befestigungsmittel erfolgen. Das Befestigungsmittel kann beispielsweise als Mutter ausgebildet sein.

Möglich ist, dass die wenigstens zwei Haltemittel einen im Wesentlichen runden Umfang aufweisen und/oder je eine vorzugsweise umlaufende Nut aufweisen. Das Werkzeug kann so in das Implantat eingreifen und gehalten werden. Dies ermöglicht ein einfaches Einbringen des Implantates.

Eine vorteilhafte Weiterbildung kann vorsehen, dass der Sockel wenigstens ein Positioniermittel umfasst. Dies kann eine Orientierungsmöglichkeit bei einem Ansetzen des Werkzeugs an das Implantat bereitstellen. Fehlerhaftem Einbringen des Implantates kann dadurch entgegengewirkt werden.

Möglich ist auch, dass der Sockel eine derartig ausgesparte Fläche aufweist, dass ein Werkstück, insbesondere ein Implantat, formschlüssig innerhalb der Fläche führbar und/oder haltbar ist. So kann das Einbringen des Implantates vereinfacht und beschleunigt werden.

Die Verbindung von Gewebe und Knochen erfolgt bei dem erfindungsgemäßen System dabei insbesondere über die Klemmfläche des Implantates. Somit erfolgt ein flächiges Verbinden von Gewebe mit Knochen bzw. ein Anpressen des Gewebes auf den Knochen. In Kombination mit einem Werkzeug, bei welchem ein Mitnahmeprofil einen Sockel und wenigstens zwei Haltemittel umfasst, wird ein Halt des Werkzeugs an einem Implantat und zudem eine Positionierung des Werkzeugs bzw. des Implantates ermöglicht. Somit kann das Werkzeug für ein besonders zeiteffizientes Einbringen eines Implantats zur Verbindung von Gewebe mit Knochen herangezogen werden. Der benötigte Zeitaufwand kann dadurch bei einem operativen Eingriff bzw. bei einem Einbringen des Implantates signifikant reduziert werden, da auf die Anbringung von Fadenmaterial verzichtet werden kann. Befestigungsmittel am Implantat ermöglichen ausreichenden Halt des Implantates.

Es kann auch vorgesehen sein, dass das Implantat zumindest einen Ausleger umfasst, der wenigstens eine Öffnung umfasst, in den zumindest ein Nagel derart einführbar ist, dass das Implantat an dem Knochen befestigbar ist. Ein zusätzliches Befestigungsmittel ermöglicht ein stabileres Befestigen des Implantates mit dem Knochen.

Im Sinne der Erfindung kann der Nagel auch als Anker bezeichnet und aufgefasst werden. Derartige Befestigungsmittel sind kostengünstig produzierbar und ermöglichen eine stabile Verbindung zwischen Knochen und Implantat.

Erfindungsgemäß kann somit auch vorgesehen sein, dass das System ein Implantat, ein Werkzeug und einen Nagel umfasst. Insofern kann vorgesehen sein, dass das System zumindest dreiteilig ausgebildet ist.

Nachfolgend wird ein Ausführungsbeispiel der Erfindung anhand von Zeichnungen näher erläutert. Hierbei zeigt:
- Fig. 1:: ein erfindungsgemäßes Implantat in einer perspektivischen Darstellung;
- Fig. 2:: ein erfindungsgemäßes Implantat in einer weiteren perspektivischen Darstellung;
- Fig. 3:: ein erfindungsgemäßes System (Werkzeug und Implantat) in einer perspektivischen Darstellung;
- Fig. 4:: eine weitere schematische Darstellung des Systems (Werkzeug und Implantat);
- Fig. 5:: eine schematische Darstellung des Werkzeugs aus Fig. 3;
- Fig. 6:: eine schematische Darstellung eines Ausschnitts des Werkzeugs aus Fig. 3;
- Fig. 7:: eine schematische Darstellung des Implantates zur Verbindung von Gewebe und Knochen;
- Fig. 8:: eine weitere schematische Darstellung des Implantates zur Verbindung von Gewebe und Knochen;
- Fig. 9:: eine schematische Darstellung einer weiteren Ausführungsform des erfindungsgemäßen Implantates;
- Fig. 10:: eine weitere Darstellung des Implantates aus Fig. 9 in einer Draufsicht;
- Fig. 11:: eine schematische Darstellung einer Ausführungsform des Nagels.

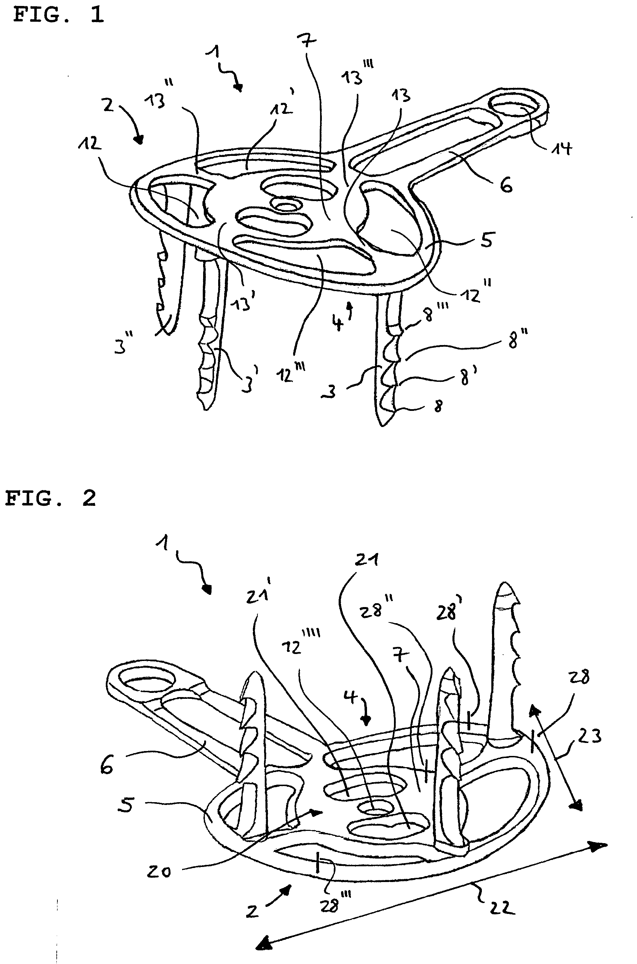

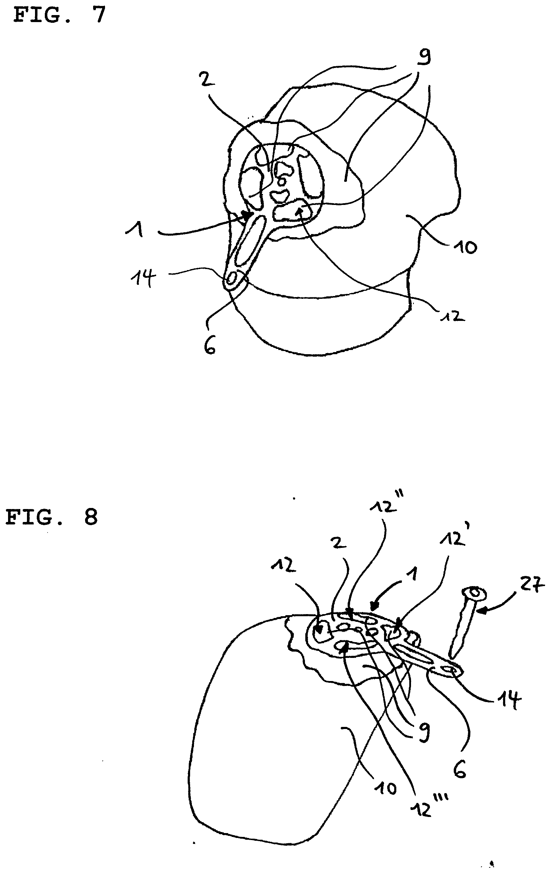

In der Fig. 1 ist eine perspektivische Darstellung eines erfindungsgemäßen Implantates 1 gezeigt. Mit der Klemmfläche 2 des Implantates 1 wird Gewebe, z.B. Sehnengewebe auf einem Knochen befestigt (siehe Fig. 7 bzw. Fig. 8). Die Klemmfläche 2 weist einen zumindest abschnittsweise gerundeten Umfang auf. Zudem ist die Klemmfläche 2 zumindest abschnittsweise gewölbt. Die Klemmfläche 2 bildet eine Ebene.
Das Implantat 1 umfasst eine Klemmfläche 2 und drei Befestigungsmittel 3, 3', 3", die jeweils einstückig an der Klemmfläche 2 angeformt sind. Dargestellt ist, dass die Befestigungsmittel 3, 3', 3" an einer Unterseite 4 der Klemmfläche 2 angeformt sind. Die Befestigungsmittel 3, 3', 3" sind somit an ihren, der Klemmfläche 2 abgewandten Seite kegelförmig ausgebildet. Dadurch können die Befestigungsmittel 3, 3', 3" besonders wirksam in ein Gewebe bzw. in einen Knochen eingebracht werden. Weiterhin ist dargestellt, dass die Befestigungsmittel 3, 3', 3" Widerhaken 8, 8', 8" bzw. 8"'. umfassen. Diese sind dazu vorgesehen, ein Lösen des Implantates 1 nach der Verbindung von Gewebe und Knochen zu verhindern.

Die Klemmfläche 2 umfasst einen äußeren Rand 5 und einen inneren Rand 7, wobei der äußere Rand 5 zumindest teilweise mittels Verbindungsstegen 13, 13', 13", 13'" zumindest teilweise mit dem inneren Rand 7 verbunden ist.

Weiterhin umfasst das Implantat 1 innerhalb der Klemmfläche 2 angeordnete Öffnungen 12 bis 12"". Die Öffnungen 12, 12', 12", 12"', 12"" sind mittels Verbindungsstegen 13, 13', 13", 13'" zumindest mit einem Teilbereich des äußeren Randes 5 der Klemmfläche 2 verbunden. Bei einer flächigen Verbindung von Implantat 1 mit Gewebe bzw. Knochen ermöglichen die Öffnungen 12, 12", 12"', 12"" eine Durchblutung des Gewebes. Nekrotischen Prozessen wird somit entgegengetreten.

Die Klemmfläche 2 umfasst weiterhin einen Ausleger 6, der eine Öffnung 14 aufweist. Durch diese Öffnung 14 kann ein Nagel (siehe Fig. 7) oder dergleichen Haltemittel geführt werden, um das Implantat 1 im Bereich dieser Öffnung 14 mit einem Knochen bzw. mit Gewebe zu verbinden. Das Implantat 1 kann dadurch besonders stabil mit Gewebe bzw. Knochen verbunden werden und ermöglicht somit eine stabile Verbindung zwischen Gewebe und Knochen. Der Ausleger 6 ist etwa entlang einer Quererstreckung 23 der Klemmfläche 2 angeordnet. Zudem sind der Ausleger 6 und die Klemmfläche 2 einstückig ausgestaltet. Dargestellt ist zudem die Längserstreckung 22 der Klemmfläche 2.

In der Fig. 2 ist eine weitere perspektivische Darstellung des Implantates 1 aus Fig. 1 gezeigt, wobei ersichtlich ist, dass die Klemmfläche 2 etwa in Ihrem Mittelpunkt eine weitere Öffnung 12"'" umfasst.

Ferner ist dargestellt, dass die Klemmfläche 2 eine Werkzeugangriffsfläche 20 umfasst. Dadurch kann das Implantat besonders einfach und unter vermindertem Zeitaufwand implantiert werden.

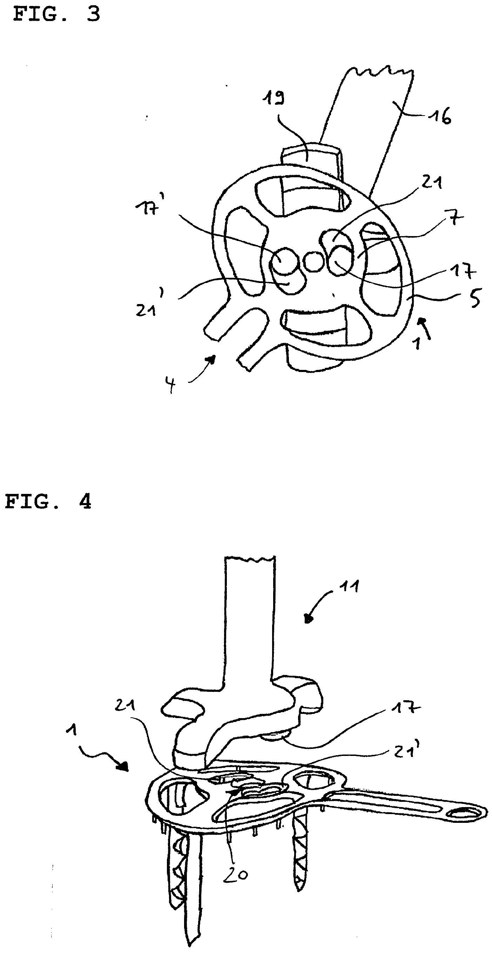

Die Werkzeugangriffsfläche kann in Form von Langlöchern 21, 21' ausgeführt sein. Die Langlöcher können dabei analog den Öffnungen 12 bis 12"" aufgefasst werden (siehe Fig. 1) und nekrotische Prozesse unterbinden. Im Ausführungsbeispiel sind die Langlöcher 21, 21' als sich jeweils konisch verjüngende Langlöcher (21, 21') ausgebildet, die derart diametral angeordnet sind, dass ein Werkzeug (siehe Fig. 3) wenigstens mittels einer Drehbewegung einbringbar und/oder ausbringbar ist. Von besonderer Bedeutung ist hierbei, dass ein Werkzeug derart mittels einer Drehbewegung ausführbar ist, dass das Implantat 1 seine Position nicht verändert, d.h. nicht durch beispielsweise eine Drehbewegung des Werkzeuges herausgedreht wird.

Die Langlöcher 21, 21' sind im Wesentlichen nierenförmig ausgebildet. Die Langlöcher 21, 21' können mit einem nicht dargestellten Werkzeug bajonettartig zusammenwirken.

Weiterhin ist dargestellt, dass die Klemmfläche 2 Fixiermittel 28, 28', 28", 28"' und 28"" umfasst. Somit umfasst die Klemmfläche 2 wenigstens ein Fixiermittel 28. Das wenigstens eine Fixiermittel 28 ist bevorzugt an der Unterseite 4 der Klemmfläche 2 angeordnet und vorzugsweise zylindrisch ausgestaltet. Das wenigstens eine Fixiermittel 28 kann insbesondere das Gewebe fixieren und somit ein Verbinden bzw. Befestigen von Gewebe und Knochen vereinfachen bzw. die Verbindung von Gewebe und Knochen verstärken. Weiterhin kann das wenigstens eine Fixiermittel 28 einen verbesserten Halt der Klemmfläche 2 mit Gewebe ermöglichen.

Es kann vorgesehen sein, dass das wenigstens eine Fixiermittel 28 an einem äußeren Rand 5 und/oder an einem inneren Rand 7 der Klemmfläche 2 angeordnet ist. Dadurch kann die Verbindung von Klemmfläche 2 und Gewebe zielgerichtet verstärkt werden.

Es kann weiterhin vorgesehen sein, dass das wenigstens eine Fixiermittel vorzugsweise einstückig an der Klemmfläche 2 angeformt ist. Dadurch kann die Stabilität der Fixiermittel 28 erhöht werden.

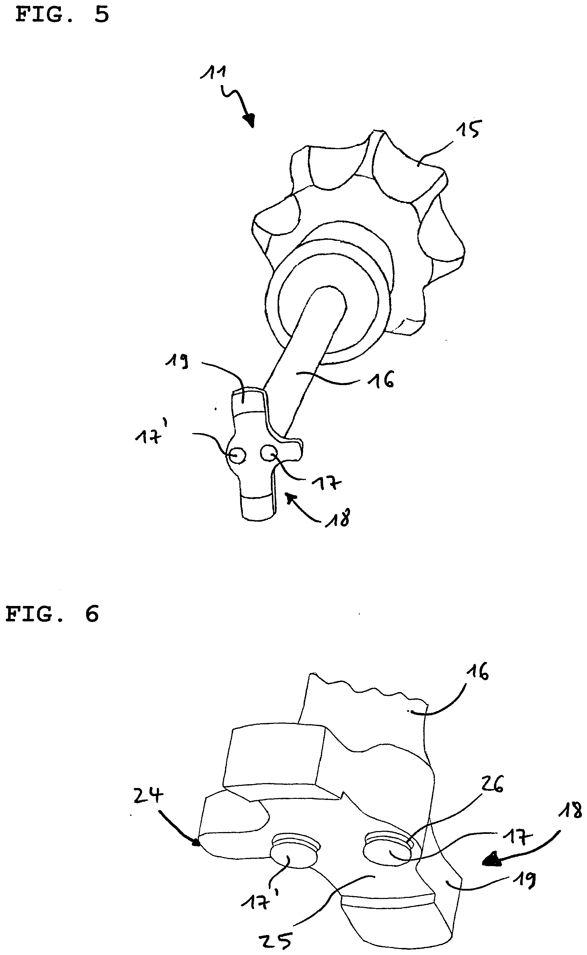

Fig. 3 zeigt ein erfindungsgemäßes System zum Verbinden von Gewebe (nicht dargestellt) mit Knochen (nicht dargestellt), umfassend ein Implantat 1 und ein Werkzeug 11. Es ist ein Ausschnitt einer Anordnung des Implantates 1 aus Fig. 1 in Kombination mit dem Werkzeug 11 gemäß Fig. 5 gezeigt. Das Implantat 1 ist in der Fig. 3 von der Unterseite 4 dargestellt.

Es ist aus der Fig. 3 ersichtlich, dass zwei Haltemittel 17, 17', die am Sockel 19 des Werkzeugs, der mit dem Schaft 16 verbunden ist, angeordnet sind, mit je konisch verjüngenden Langlöchern 21, 21' am Implantat 1 bzw. an dessen Klemmfläche 2 zusammenwirken. Die konischen Verjüngungen der Langlöcher 21, 21' sind hierbei derart diametral angeordnet, dass die Haltemittel 17, 17' bzw. das Werkzeug 11 mittels einer Drehbewegung einbringbar und/oder ausbringbar ist. Ein Werkzeug 11 kann somit bajonettartig mit der Klemmfläche 2 des Implantates 1 zusammenwirken. Die Klemmfläche 2 weist daher eine Werkzeugangriffsfläche 20 auf (siehe auch Fig. 4).

In Fig. 4 ist eine weitere schematische Darstellung des Systems, umfassend ein Werkzeug 11 und ein Implantat 1 gezeigt. Das Haltemittel 17 des Werkzeugs 11 kann mit der Werkzeugangriffsfläche 20 des Implantates 1 zusammenwirken und so ein Einbringen des Implantates 1, beispielsweise in einen menschlichen Körper, auf besonders einfache Art und Weise ermöglichen.

Ein bajonettartiges Zusammenwirken von Werkzeugangriffsfläche 20 (mittels Langlöcher 21, 21') und Werkzeug 11 (mittels Haltemittel 17, 17') ermöglicht zudem nach einem Einbringen des Implantates, beispielsweise in einen menschlichen Körper, dass das Werkzeug 11 danach besonders einfach von dem Implantat 1 entfernt bzw. getrennt werden kann.

Hierbei ist von besonderem Vorteil, dass bei einem Entfernen des Werkzeugs 11 die Position des Implantates 1 bzw. eine stabile Befestigung von Gewebe mit Knochen durch das Implantat 1 nicht beeinträchtigt wird.

Fig. 5 zeigt ein Werkzeug 11 in einer perspektivischen Darstellung. Das Werkzeug 11 ist zum Anbringen des Implantats 1 zur Verbindung von Gewebe mit Knochen vorgesehen. Das Werkzeug 11 umfasst ein Griffmittel 15 und einen daran drehfest angeordneten Schaft 16. Weiterhin umfasst das Werkzeug 11 ein Mitnahmeprofil 18.

Das Mitnahmeprofil 18 umfasst hierbei einen Sockel 19 und zwei Haltemittel 17, 17', die mit einer Werkzeugangriffsfläche am Implantat zusammenwirken können und so ein vereinfachtes implantieren des Implantates ermöglichen können. Die Haltemittel 17, 17' sind als Nasen ausgebildet und weisen einen im Wesentlichen runden Umfang auf.

Die Haltemittel 17, 17' sind mit dem Sockel 19 verbunden, der einen größeren Umfang aufweist als ein Umfang des Schafts 16 Zudem ist der Sockel 19 einstückig mit dem Schaft 16 verbunden. Weiterhin sind der Sockel 19 und der Schaft 16 einstückig ausgestaltet.

Fig. 6 zeigt einen vergrößerten Ausschnitt des Werkzeugs aus Fig. 5, wobei ersichtlich ist, dass der Sockel 19 eine derartig ausgesparte Fläche 25 aufweist, dass ein Werkstück, insbesondere das Implantat (nicht gezeigt), formschlüssig innerhalb der Fläche 25 führbar und/oder haltbar ist.

Innerhalb der Fläche 25 sind die Haltemittel 17, 17' angeordnet, die je eine vorzugsweise umlaufende Nut 26, 26' aufweisen. Der Sockel 19 umfasst weiterhin ein Positioniermittel 24.

Erfindungsgemäß ist vorgesehen, dass die Fläche 25 des Werkzeugs 11 mit der Klemmfläche des Implantates (nicht dargestellt) formschlüssig zusammenwirkt. Auf diese Weise ist die Klemmfläche bzw. das Implantat durch das Werkzeugll bei einem Einbringen des Implantates, beispielsweise in einen menschlichen Körper, gehalten. Somit kann das Einbringen des Implantates signifikant erleichtert werden.

Fig. 7 zeigt eine schematische Darstellung des Implantates 1 zur Verbindung von Gewebe 9 und Knochen 10. Das Gewebe 9, insbesondere Sehnengewebe, wird hierbei mittels des Implantates 1 auf einem Knochen angepresst. Dazu umfasst das Implantat 1 eine Klemmfläche 2. Die Klemmfläche 2 ermöglicht somit ein Befestigen bzw. ein Verbinden des Gewebes 9 mit Knochen 10 ohne Verwendung eines Fadens oder dergleichen. Zur Vermeidung eines unbeabsichtigten Lösens des Implantates 1 umfasst dieses Befestigungsmittel (nicht dargestellt), die an der Klemmfläche 2 angeordnet sind (siehe Fig. 1).

Bei einem Aufbringen und Anpressen des Implantates 1 auf Gewebe 9 wird Gewebe 9 durch Öffnungen 12 gepresst. Unter Umständen kann das Gewebe 9 auch wulstartig durch die Öffnungen 12 hervorstehen. Gewebe wird durch die Öffnungen 12, sowie die Langlöcher 21, 21a (siehe z.B. Fig. 1) der Klemmfläche 2 des Implantates 1 nicht bedeckt. Dadurch wird ermöglicht, dass das Gewebe 9 ausreichend durchblutet werden kann und nicht absterben kann, d.h. dass nekrotische Prozesse unterbunden werden bzw. minimiert werden.

Insofern wird das Gewebe 9 nicht vollständig durch die Klemmfläche 2 des Implantates 1 bedeckt. Gleichzeitig wird jedoch durch flächiges Anpressen des Implantates 1 bzw. der Klemmfläche 2 eine Fixierung des Gewebes 9 und eine stabile Verbindung des Gewebes 9 mit Knochen 10 ermöglicht. Dies wird auch durch einen inneren Rand und einen äußeren Rand der Klemmfläche 2 (Fig. 1) ermöglicht.

In der Fig. 8 ist schematisch dargestellt, dass die Befestigung des Implantates 1 an einem Knochen 10 unter Verwendung eines Nagels 27 erfolgen kann. Der Nagel 27 kann in einer Öffnung 14 im Ausleger 6 des Implantates 1 angeordnet sein. Es ist weiterhin möglich, dass nach einem Einbringen des Nagels 27 der Ausleger 6 gebogen wird, um mit dem Knochen 10 verbunden zu werden. Hierzu kann vorgesehen sein, dass der Ausleger 6 an einem Übergangsbereich zu der Klemmfläche 2 eine verminderte Materialdicke aufweist als die Klemmfläche 2. Somit kann der Ausleger 6 in diesem Bereich, der als Schwächung bezeichnet werden kann, besonders einfach gebogen werden. Dadurch können individuelle anatomische Gegebenheiten berücksichtigt werden.

Der Nagel 27 kann als weiteres Befestigungsmittel aufgefasst werden, das nicht einstückig mit der Klemmfläche 2 verbunden ist. Durch Verwenden eines Nagels 27 kann das Implantat 1 zusätzlich beispielsweise an einem Knochen 10 befestigt werden. Dies wirkt sich vorteilhaft auch für die Verbindung von Gewebe 9 und Knochen 10 aus. Das Implantat 1 kann somit als Sehnen-Fixationsplatte aufgefasst werden.

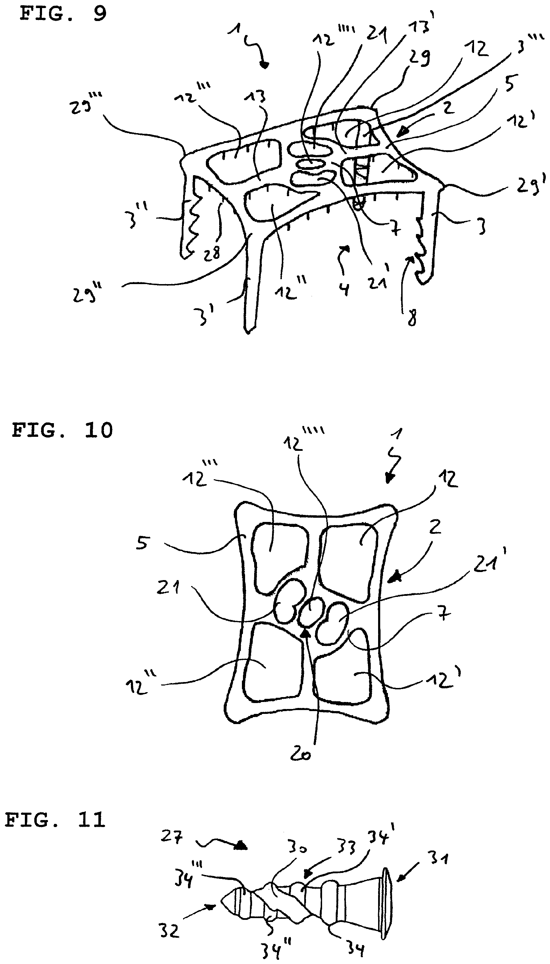

In den Fig. 9 und 10 ist eine weitere Ausführungsform des Implantates 1 gezeigt. Das Implantat 1 umfasst eine Klemmfläche 2. Im Unterscheid zu dem in Fig. 1 dargestellten Implantat umfasst das in Fig. 9 gezeigte Implantat eine Klemmfläche 2, welche einen im Wesentlichen viereckigen Umfang aufweist. Die Klemmfläche umfasst im gezeigten Ausführungsbeispiel vier einstückig an jeweils an einer Ecke 29, 29', 29", 29'" der Klemmfläche 2 angeformte Befestigungsmittel 3, 3', 3", 3"'.

Die Klemmfläche 2 umfasst einen äußeren Rand 5 und einen inneren Rand 7, wobei der äußere Rand 5 zumindest teilweise mittels Verbindungsstegen 13, 13' zumindest teilweise mit dem inneren Rand 7 verbunden ist.

Das Implantat 1 ist im gezeigten Ausführungsbeispiel ohne Ausleger dargestellt. Es kann jedoch im Rahmen der Erfindung vorgesehen sein, dass ein zumindest ein Ausleger vorzugsweise einstückig an der Klemmfläche 2 angeformt ist.

Das Implantat 1 umfasst eine Werkzeugangriffsfläche 20, welche durch vorzugsweise zwei diametral zueinander angeordnete Langlöcher 21, 21', die sich konisch verjüngen, gebildet wird.

Die Klemmfläche 2 umfasst eine zentral angeordnete, zwischen den Langlöchern 21, 21' angeordnete Öffnung 12"'. Weiterhin umfasst die Klemmfläche 2 vier weitere, symmetrisch angeordnete Öffnungen 12, 12', 12" und 12"". Die Öffnungen 12, 12', 12", 12"', 12"" sind mittels Verbindungsstegen 13, 13' zumindest mit einem Teilbereich des äußeren Randes 5 der Klemmfläche 2 verbunden. Bei einer flächigen Verbindung von Implantat 1 mit Gewebe bzw. Knochen ermöglichen die Öffnungen 12, 12', 12", 12'", 12"" eine Durchblutung des Gewebes. Nekrotischen Prozessen wird somit entgegengetreten.

Auch die Langlöcher 21, 21' stellen sinngemäß Öffnungen dar und können nekrotische Prozesse unterbinden.

Unabhängig von der geometrischen Ausgestaltung des Implantates 1 kann ein Verhältnis der Summen der Öffnungen und ggf. der Langlöcher der Klemmfläche zu der Gesamtfläche der Klemmfläche angegeben werden. Die Öffnungen der Klemmfläche und ggf. die Langlöcher können zusammen als so genannte Aussparungen bezeichnet werden. Die Gesamtfläche der Klemmfläche wird durch ihren jeweiligen Umfang bestimmt. Etwaige Ausleger sind hiervon explizit ausgenommen.

Dabei ist vorteilhaft, wenn das Verhältnis der Aussparungen bzw. der Summe der Flächen der Öffnungen der Klemmfläche und der Langlöcher zu der Gesamtfläche der Klemmfläche 30% bis 70% beträgt. Besonders vorteilhaft ist, wenn das Verhältnis der Aussparungen bzw. der Summe der Flächen der Öffnungen der Klemmfläche und der Langlöcher zu der Gesamtfläche der Klemmfläche 40% bis 60% beträgt.

Erfindungsgemäß kann auch vorgesehen sein, dass etwa 50% der Gesamtfläche der Klemmfläche Öffnungen aufweisen. Dies kann Langlöcher mit einschließen.

Es kann somit ein optimiertes Verhältnis zwischen Öffnungen und Gesamtfläche bereitgestellt werden, d.h. ein optimiertes Verhältnis zwischen Auflagefläche und somit Befestigung des Implantates sowie Öffnungen bzw. Ermöglichen von Durchblutung des Gewebes und somit Minimierung nekrotischer Prozesse.

Fig. 10 zeigt die symmetrische Anordnung aller Öffnungen 12, 12', 12", 12'" sowie die symmetrische Anordnung der Öffnung 12"" zentriert in der Klemmfläche 2. Weiterhin ist die symmetrische Anordnung der Langlöcher 21, 21' bezogen auf die Öffnung 12"" dargestellt.

Fig. 11 zeigt eine schematische Darstellung einer Ausführungsform des Nagels 27. Der Nagel 27 umfasst einen Nagelschaft 33. Der Nagel 27 weist an einem Ende ein Kopfstück 31 und an einem anderen Ende eine Verjüngung 32 auf. Die Verjüngung 32 ist dabei konisch ausgebildet. Dadurch kann der Nagel 27 vereinfacht in einen Knochen eingetrieben werden. Das Kopfstück 31 kann mit dem Werkzeug (nicht gezeigt) formschlüssig zusammenwirken. Zum weiterhin vereinfachten Eintreiben des Nagels 27 weist dieser im Bereich des Nagelschafts 33 eine vorzugsweise umlaufende Spiralnut 30 auf (etwa 1 Gang / 360° auf einer Länge des Nagels 27 von 10 mm). Der Nagel 27 weist im Ausführungsbeispiel eine Gesamtlänge von etwa 13 bis 14 mm auf. Der Nagelschaft 33 weist im Ausführungsbeispiel eine Gesamtlänge von etwa 10 mm auf. Der Nagel 27 ist im Ausführungsbeispiel konisch ausgebildet und weist im Bereich der Verjüngung 32 eine Dicke von etwa 1,5 mm auf. Die Dicke im Bereich des Kopfstücks 31 beträgt etwa 2,0 bis 2,1 mm.

Weiterhin umfasst der Nagelschaft 33 mehrere Ringwulste 34, 34', 34" und 34'". Diese ermöglichen in Kombination mit der Ausbildung der Spiralnut 30 ein vereinfachtes Eintreiben des Nagels 27 und einen sicheren Halt, beispielsweise in einem Knochen.

Der Nagel 27 kann erfindungsgemäß Bestandteil eines Systems, umfassend ein Werkzeug und ein Implantat, darstellen (siehe Fig. 4).

### Bezugszeichenliste:

- 1: Implantat
- 2: Klemmfläche
- 3: Befestigungsmittel
- 3': Befestigungsmittel
- 3": Befestigungsmittel
- 3'": Befestigungsmittel
- 4: Unterseite (der Klemmfläche)
- 5: Äußerer Rand
- 6: Ausleger
- 7: Innerer Rand
- 8: Widerhaken
- 8'-8'": Widerhaken
- 9: Gewebe
- 10: Knochen
- 11: Werkzeug
- 12: Öffnung (der Klemmfläche)
- 12'-12"'": Öffnung (der Klemmfläche)
- 13: Verbindungssteg
- 13'-13'": Verbindungssteg
- 14: Öffnung (des Auslegers)
- 15: Griffmittel
- 16: Schaft
- 17, 17': Haltemittel
- 18: Mitnahmeprofil
- 19: Sockel
- 20: Werkzeugangriffsfläche
- 21: Langloch
- 22: Längserstreckung (der Klemmfläche)
- 23: Quererstreckung (der Klemmfläche)
- 24: Positioniermittel
- 25: Fläche
- 26, 26': Nut
- 27: Nagel
- 28: Fixiermittel
- 28'-28"": Fixiermittel
- 29'-29'": Ecke (der Klemmfläche)
- 30: Spiralnut
- 31: Kopfstück
- 32: Verjüngung
- 33: Nagelschaft
- 34-34'": Ringwulst

## Patentansprüche

1. Implantat (1) zur flächigen Verbindung von Gewebe (9) mit Knochen (10), wobei das Implantat (1) eine Klemmfläche (2) und wenigstens drei mit der Klemmfläche (2) verbundene Befestigungsmittel (3, 3', 3") umfasst, wobei die Klemmfläche (2) einen äußeren Rand (5) und einen inneren Rand (7) umfasst, wobei der äußere Rand (5) zumindest teilweise mittels wenigstens eines Verbindungssteges (13) zumindest teilweise mit dem inneren Rand (7) verbunden ist und/oder dass die Klemmfläche (2) einen äußeren Rand (5) und wenigstens eine innerhalb der Klemmfläche (2) angeordnete Öffnung (12) umfasst, welche Öffnung (12) mittels wenigstens eines Verbindungssteges (13) zumindest mit einem Teilbereich des äußeren Randes (5) der Klemmfläche (2) verbunden ist,
**dadurch gekennzeichnet, dass**
dass die Klemmfläche (2) wenigstens einen Ausleger (6) umfasst, der wenigstens eine Öffnung (14) aufweist und dass die Klemmfläche (2) wenigstens ein Fixiermittel (28) umfasst, das an einer Unterseite (4) angeordnet ist.

2. Implantat nach Anspruch 1, **dadurch gekennzeichnet, dass** wenigstens ein Befestigungsmittel (3, 3', 3") einstückig an der Klemmfläche (2) angeformt ist und/oder dass zumindest ein Befestigungsmittel (3, 3', 3") kegelförmig ausgebildet ist und/oder dass das wenigstens eine Befestigungsmittel (3, 3', 3") wenigstens einen Widerhaken (8)umfasst.

3. Implantat nach wenigstens einem der vorhergehenden Ansprüche, **dadurch gekennzeichnet, dass** wenigstens zwei Befestigungsmittel (3, 3', 3") in etwa parallel zueinander angeordnet sind und/oder unterschiedliche Längen aufweisen und/oder dass die Klemmfläche (2) eine Ebene bildet, wobei wenigstens ein Befestigungsmittel (3, 3', 3") in etwa senkrecht zu der Ebene und/oder wenigstens ein Befestigungsmittel (3, 3', 3") in etwa parallel zu der Ebene angeordnet ist.

4. Implantat nach wenigstens einem der vorangehenden Ansprüche, **dadurch gekennzeichnet, dass** die Klemmfläche (2) eine Werkzeugangriffsfläche (20) umfasst.

5. Implantat nach wenigstens einem der vorangehenden Ansprüche, **dadurch gekennzeichnet, dass** der wenigstens eine Ausleger (6) etwa entlang einer Quererstreckung (23) der Klemmfläche (2) angeordnet ist und/oder dass der wenigstens eine Ausleger (6) und die Klemmfläche (2) einstückig ausgestaltet sind.

6. Implantat nach wenigstens einem der vorangehenden Ansprüche, **dadurch gekennzeichnet, dass** die Klemmfläche (2) wenigstens zwei sich jeweils konisch verjüngende Langlöcher (21, 21') umfasst, die diametral angeordnet sind

7. Implantat nach wenigstens einem der vorangehenden Ansprüche, **dadurch gekennzeichnet, dass** die Klemmfläche (2) einen zumindest abschnittsweise gerundeten Umfang aufweist und/oder zumindest abschnittsweise gewölbt ist.

8. Implantat nach wenigstens einem der vorangehenden Ansprüche, **dadurch gekennzeichnet, dass** die Klemmfläche (2) einen im Wesentlichen viereckigen Umfang aufweist und vier einstückig an jeweils an einer Ecke (29, 29', 29", 29'") der Klemmfläche (2) angeformte Befestigungsmittel (3, 3', 3", 3'") umfasst.

9. System zum Verbinden von Gewebe (9) mit Knochen (10), umfassend wenigstens ein Implantat (1) gemäß Anspruch 1, und wenigstens ein Werkzeug (11), wobei das Werkzeug (11) ein Griffmittel (15) und einen daran drehfest angeordneten Schaft (16) umfasst, wobei das Werkzeug (11) ein Mitnahmeprofil (18) umfasst, wobei das Mitnahmeprofil (18) einen Sockel (19) und wenigstens zwei Haltemittel (17, 17') umfasst.

10. System nach Anspruch 9, **dadurch gekennzeichnet, dass** die wenigstens zwei Haltemittel (17, 17') mit dem Sockel (19) verbunden sind und/oder das Mitnahmeprofil (18) einen Sockel (19) umfasst, der einen größeren Umfang als ein Umfang des Schafts (16) aufweist und/oder einstückig mit dem Schaft (16) verbunden ist.

11. System nach Anspruch 9 oder 10, **dadurch gekennzeichnet, dass** der Sockel (19) und der Schaft (16) einstückig ausgestaltet sind und/oder die wenigstens zwei Haltemittel (17, 17') vorzugsweise als Nasen ausgebildet sind und einen im Wesentlichen runden oder polygonen Umfang aufweisen.

12. System nach wenigstens einem der vorangehenden Ansprüche 9 bis 11, **dadurch gekennzeichnet, dass** die wenigstens zwei Haltemittel (17, 17') einen im Wesentlichen runden Umfang aufweisen und/oder je eine vorzugsweise umlaufende Nut (26, 26') aufweisen und/oder dass der Sockel (19) wenigstens ein Positioniermittel (24) umfasst.

## Claims

1. Implant (1) for planar connection of tissue (9) to bone (10), wherein the implant (1) comprises a clamping surface (2) and at least three securing means (3, 3', 3") connected to the clamping surface (2), wherein the clamping surface (2) comprises an outer edge (5) and an inner edge (7), wherein the outer edge (5) is connected at least partially to the inner edge (7) at least partially by means of at least one connecting web (13), and/or the clamping surface (2) comprises an outer edge (5) and at least one opening (12) arranged within the clamping surface (2), which opening (12) is connected by means of at least one connecting web (13) at least to a partial region of the outer edge (5) of the clamping surface (2),
**characterized in that**
the clamping surface (2) comprises at least one projection (6), which has at least one opening (14), and the clamping surface (2) comprises at least one fixing means (28), which is arranged on an underside (4) .

2. Implant according to Claim 1, **characterized in that** at least one securing means (3, 3', 3") is formed integrally on the clamping surface (2), and/or **in that** at least one securing means (3, 3', 3") is cone-shaped, and/or **in that** the at least one securing means (3, 3', 3") comprises at least one barb (8).

3. Implant according to at least one of the preceding claims, **characterized in that** at least two securing means (3, 3', 3") are arranged approximately parallel to each other and/or have different lengths, and/or **in that** the clamping surface (2) forms a plane, wherein at least one securing means (3, 3', 3") is arranged approximately perpendicular to the plane and/or at least one securing means (3, 3', 3") is arranged approximately parallel to the plane.

4. Implant according to at least one of the preceding claims, **characterized in that** the clamping surface (2) comprises a tool engagement surface (20).

5. Implant according to at least one of the preceding claims, **characterized in that** the at least one projection (6) is arranged approximately along a transverse extent (23) of the clamping surface (2), and/or **in that** the at least one projection (6) and the clamping surface (2) are formed in one piece.

6. Implant according to at least one of the preceding claims, **characterized in that** the clamping surface (2) comprises at least two oblong holes (21, 21') which each taper conically and are arranged diametrically.

7. Implant according to at least one of the preceding claims, **characterized in that** the clamping surface (2) has an at least partially rounded circumference and/or is at least partially curved.

8. Implant according to at least one of the preceding claims, **characterized in that** the clamping surface (2) has a substantially rectangular circumference and comprises four securing means (3, 3', 3", 3"') formed integrally at respective corners (29, 29', 29", 29"') of the clamping surface (2).

9. System for connecting tissue (9) to bone (10), comprising at least one implant (1) according to Claim 1 and at least one tool (11), wherein the tool (11) comprises a gripping means (15) and a shaft (16) arranged thereon for conjoint rotation, wherein the tool (11) comprises a driving profile (18), wherein the driving profile (18) comprises a base (19) and at least two holding means (17, 17').

10. System according to Claim 9, **characterized in that** the at least two holding means (17, 17') are connected to the base (19), and/or the driving profile (18) comprises a base (19) which has a greater circumference than a circumference of the shaft (16) and/or is integrally connected to the shaft (16).

11. System according to Claim 9 or 10, **characterized in that** the base (19) and the shaft (16) are formed in one piece, and/or the at least two holding means (17, 17') are preferably designed as lugs and have a substantially round or polygonal circumference.

12. System according to at least one of Claims 9 to 11, **characterized in that** the at least two holding means (17, 17') have a substantially round circumference and/or each have a preferably circumferential groove (26, 26'), and/or **in that** the base (19) comprises at least one positioning means (24).

## Revendications

1. Implant (1) destiné à une liaison à plat entre des tissus (9) et des os (10), l'implant (1) comprenant une surface de serrage (2) et au moins trois moyens de fixation (3, 3', 3") reliés à la surface de serrage (2), dans lequel la surface de serrage (2) comprend un bord extérieur (5) et un bord intérieur (7), le bord extérieur (5) étant relié au moins en partie au bord intérieur (7) à l'aide au moins en partie d'au moins une barrette de liaison (13), et/ou la surface de serrage (2) comprenant un bord extérieur (5) et au moins un orifice (12) disposé à l'intérieur de la surface de serrage (2), ledit orifice (12) étant relié à l'aide d'au moins une barrette de liaison (13) au moins à une zone partielle du bord extérieur (5) de la surface de serrage (2),
**caractérisé en ce que** la surface de serrage (2) comprend au moins un bras (6) qui présente au moins un orifice (14), et **en ce que** la surface de serrage (2) comprend au moins un moyen de fixation (28) qui est disposé sur une face inférieure (4).

2. Implant selon la revendication 1, **caractérisé en ce qu'**au moins un moyen de fixation (3, 3', 3") est rapporté intégralement à la surface de serrage (2), et/ou **en ce qu'**au moins un moyen de fixation (3, 3', 3") est réalisé de manière conique, et/ou **en ce que** ledit au moins un moyen de fixation (3, 3', 3") comprend au moins une barbe (8).

3. Implant selon l'une quelconque des revendications précédentes, **caractérisé en ce qu'**au moins deux moyens de fixation (3, 3', 3") sont disposés approximativement en parallèle les uns aux autres, et/ou présentent des longueurs différentes, et/ou **en ce que** la surface de serrage (2) constitue un plan, au moins un moyen de fixation (3, 3', 3") étant disposé de manière approximativement perpendiculaire au plan et/ou au moins un moyen de fixation (3, 3', 3") étant disposé approximativement en parallèle au plan.

4. Implant selon au moins l'une des revendications précédentes, **caractérisé en ce que** la surface de serrage (2) comprend une surface de prise d'outil (20).

5. Implant selon au moins l'une des revendications précédentes, **caractérisé en ce que** ledit au moins un bras (6) est disposé approximativement le long d'une extension transversale (23) de la surface de serrage (2), et/ou **en ce que** ledit au moins un bras (6) et la surface de serrage (2) sont conçus intégralement.

6. Implant selon au moins l'une des revendications précédentes, **caractérisé en ce que** la surface de serrage (2) comprend au moins deux trous oblongs (21, 21') s'amincissant de manière conique respectivement et qui sont disposés diamétralement.

7. Implant selon au moins l'une des revendications précédentes, **caractérisé en ce que** la surface de serrage (2) présente une circonférence arrondie au moins par endroits et/ou courbe au moins par endroits.

8. Implant selon au moins l'une des revendications précédentes, **caractérisé en ce que** la surface de serrage (2) présente une circonférence substantiellement rectangulaire et comprend quatre moyens de fixation (3, 3', 3", 3"') rapportés intégralement dans un coin (29, 29', 29", 29"') de la surface de serrage (2) respectivement.

9. Système permettant de relier des tissus (9) et des os (10), comprenant au moins un implant (1) selon la revendication 1 et au moins un outil (11), l'outil (11) comprenant un moyen de préhension (15) et une tige (16) disposée de manière verrouillée en rotation sur celui-ci, l'outil (11) comprenant un profil d'entraînement (18), le profil d'entraînement (18) comprenant une base (19) et au moins deux moyens de retenue (17, 17').

10. Système selon la revendication 9, **caractérisé en ce que** les au moins deux moyens de retenue (17, 17') sont reliés à la base (19) et/ou le profil d'entraînement (18) comprend une base (19) qui présente une circonférence supérieure à une circonférence de la tige (16) et/ou est reliée intégralement à la tige (16) .

11. Système selon la revendication 9 ou 10, **caractérisé en ce que** la base (19) et la tige (16) sont conçues intégralement et/ou les au moins deux moyens de retenue (17, 17') sont de préférence réalisés sous forme de becs et présentent une circonférence substantiellement ronde ou polygonale.

12. Système selon au moins l'une des revendications précédentes 9 à 11, **caractérisé en ce que** les au moins deux moyens de retenue (17, 17') présentent une circonférence substantiellement ronde et/ou respectivement une rainure (26, 26') de préférence périphérique, et/ou **en ce que** la base (19) comprend au moins un moyen de positionnement (24).
